# EUROPEAN PATENT APPLICATION

(11) **EP 4 744 682 A1**
(43) Date of publication of application: **20.05.2026**
(21) Application number: 24840129.1
(22) Date of filing: 12.07.2024
(51) Int. Cl.: A61K 47/69, A61K 33/24, A61P 29/00, A61P 37/00, A61P 1/00, A61P 25/00

(54) **NOVEL CERIUM OXIDE NANOCOMPOSITE WITH ENHANCED BIOSTABILITY AND USE THEREOF**

(30) Priority: 13.07.2023 KR 20230091149
(71) Applicant: Cenyx Biotech Inc., Seoul 04781 (KR)
(72) Inventor: CHA, Bong Geun, Hanam-si Gyeonggi-do 12926 (KR); PARK, Joongpill, Suwon-si Gyeonggi-do 16320 (KR); YOON, Yonghee, Seoul 03730 (KR)
(74) Representative: V.O.
(86) International application number: PCT/KR2024/010018
(87) International publication number: WO 2025/014319

(57) **Abstract**

The present invention relates to a cerium oxide nanocomplex, a method for preparing the same, and a composition for preventing or treating inflammatory or autoimmune diseases comprising the cerium oxide nanocomplex as an active ingredient. The present invention may be used as an excellent therapeutic composition where both biostability and *in vivo* reactive oxygen species-scavenging efficiency are maximized by modifying the surface of cerium oxide nanoparticles with a pyrrolidone-derived polymer at an optimal content. In addition, the method for preparing the nanocomplex of the present invention can produce uniform particles having an optimal diameter while completely removing reaction residues and nitrate-derived toxicity through a simple process of washing with a sodium chloride solution of an indicated concentration. The nanocomplex of the present invention significantly suppresses inflammatory responses in various tissues. It is particularly can be advantageously applied as an excellent therapeutic composition that minimizes neuronal damage caused by excessive inflammatory responses in the peri-hematomal region following intracerebral hemorrhage, thereby restoring neurological function and significantly improving patient survival.

## Description

### Technical Field

The present disclosure relates to a cerium oxide nanocomplex with significantly improved biostability despite a simplified production process, and to a composition for preventing or treating various inflammatory diseases containing the cerium oxide nanocomplex as an active ingredient.

### Background Art

Recently, extensive research has been conducted on nanoparticle-based therapeutics as pharmacological agents for the treatment of various intractable diseases. Nanoparticles are being studied in diverse medical fields such as imaging diagnostics, drug delivery systems, and therapeutics, based on their unique optical, electromagnetic, and chemical properties arising from their nanoscale diameters. In particular, based on their intrinsic abilities such as magnetically induced heat generation and antioxidant activity, nanoparticles can themselves serve as active pharmacological components. Moreover, by tailoring the composition, shape, size, and surface characteristics of the particles to suit specific purposes, significant efforts have been directed toward developing nanoparticles that exhibit optimized properties suitable for a wide range of medical applications.

Reactive oxygen species (ROS) are natural byproducts of oxygen metabolism which are essential for life due to their functions such as cellular signaling and immune responses. However, excessively generated ROS induce oxidative damage to biomolecules and cells, causing various forms of oxidative stress. Cerium oxide nanoparticles, also known as ceria nanoparticles, exhibit thermal stability at high temperatures. Their lattice structure enables redox reactions between Ce⁴⁺ and Ce³⁺ depending on the surrounding oxygen concentration. Consequently, they may be applied as electrolytes in solid-state batteries, materials for UV filters, oxygen sensors, and optical devices.

In the medical field, cerium oxide nanoparticles are also gaining attention as therapeutic compositions for a wide range of inflammatory diseases. Cerium oxide nanoparticles reversibly bind oxygen, and the change in oxidation state between Ce³⁺ and Ce⁴⁺ on the particle surface enables efficient removal of free radicals. This not only effectively controls secondary brain damage caused by neuroinflammation arising from various etiologies but also function as a pharmacological agent for a wide range of diseases driven by excessive inflammatory responses in tissues such as the skin, intestine, lung, and liver (for example, dermatitis, inflammatory bowel disease, Crohn's disease, acute respiratory distress syndrome, inflammatory liver diseases, and the like). However, due to the fine diameter characteristic of nanoparticles, suppressing aggregation during mass production while maintaining uniform particle size, excellent dispersibility, and low surface charge (Zeta-potential) is a significant challenge. Accordingly, there is a demand for developing cerium oxide nanoparticles having a novel structure that exhibit excellent antioxidant effects and optimized particle properties, even when produced on an industrial scale aimed at securing therapeutically effective amount.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made intensive studies to develop a nanoparticle-based therapeutic composition capable of efficiently treating various inflammatory diseases caused by oxidative stress-derived tissue damage. As a result, it was found that when a pyrrolidone polymer of the formula 1 below is bound to the surface of cerium oxide nanoparticles at a specific concentration to form an outer layer, the dispersion of the nanoparticles is significantly improved and the surface charge converges to 0 mV. This enables the use of an outstanding therapeutic composition that maximizes both biocompatibility and the efficiency of removing reactive oxygen species within the body.

Accordingly, it is an object of the present invention to provide a novel cerium oxide nanocomplex a method for preparing the same.

It is another object of the present invention to provide a composition for preventing or treating inflammatory or autoimmune diseases, comprising the nanocomplex of the preent invention.

Other objects and advantages of the present invention will become more apparent from the following detailed description, the appended claims, and the accompanying drawings.

### Technical Solution

In one aspect of this invention, there is provided a cerium oxide nanocomplex comprising:
(a) core layer of cerium oxide nanoparticles;
(b) an outer layer comprising a polymer represented by the following Formula 1: 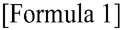 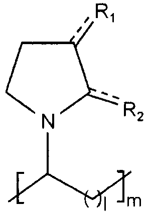 wherein R₁ and R₂ are each independently hydrogen or oxygen, - - - represents a single bond or a double bond, l is 1 or 2, and m is an integer of 100 to 1000.

The present inventors have made intensive studies to develop a nanoparticle-based therapeutic composition capable of efficiently treating various inflammatory diseases caused by oxidative stress-derived tissue damage. As a result, it was found that when a pyrrolidone polymer of above formula 1 bound to the surface of cerium oxide nanoparticles at a specific concentration to form an outer layer, the dispersion of the nanoparticles is significantly improved and the surface charge converges to 0 mV, thus may be utilized as a therapeutic composition with maximized biocompatibility and reactive oxygen species-scavenging efficiency.

As used herein, the term "core layer" refers to an innermost layer having only one surface in contact with other layers in a multilayer composite.

As used herein, the term "multilayer composite" refers to a composite consisting of a plurality of layers composed of different components, and includes, without limitation, a laminated multilayer structure, a core-shell multilayer structure, and combinations thereof. Specifically, the multilayer composite according to the present disclosure has a core-shell type multilayer structure in which nanoparticles are present at the center and the polymer of Formula 1 and a biocompatible dispersion stabilizer surround the nanoparticles on the outer shell.

As used herein, the term "outer layer" refers to a layer surrounding the core layer of the core-shell structure and located farther from the center than the core layer. The outer layer need not necessarily be the layer directly contacting the core layer; additional layers closer to the core layer than the outer layer (e.g., an inner layer) may exist. Furthermore, the outer layer need not necessarily be the outermost layer; additional outermost layers located farther from the core than the outer layer may also exist.

According to the present disclosure, the boundary between the core layer and the outer layer may or may not be clearly distinguished. If a boundary between the core layer and the outer layer is not clear, each component of the core layer and the outer layer may be mixed in the vicinity of the interface or in the entire section.

As used herein, the term "polymer" refers to a synthetic or natural polymer compound in which the same or different types of monomers are continuously linked. Thus, the polymer includes a homopolymer (a polymer in which one type of monomer is polymerized) and an interpolymer prepared by the polymerization of at least two different monomers, wherein the interpolymer includes both a copolymer (a polymer prepared from two different monomers) and a polymer prepared from more than two different monomers. Specifically, the polymer of Formula 1 used in the present disclosure is a homopolymer.

As used herein, the term "alkyl" refers to a straight or branched chain, saturated hydrocarbon group, and includes, for examples, methyl, ethyl, propyl, isopropyl, etc. C₁-C₃ alkyl refers to an alkyl group having an alkyl unit having 1 to 3 carbon atoms, and when C₁-C₃ alkyl is substituted, the number of carbon atoms in the substituent is not included.

As used herein, the term "biocompatibility" refers to a property of not causing short-term or long-term side effects when administered *in vivo* and in contact with cells, tissues or body fluids of organs, and specifically, refers to not only tissue compatibility and blood compatibility that do not cause tissue necrosis or blood coagulation in contact with biological tissue or blood, but also biodegradability, which is a property of being disappeared after a certain period of time after *in vivo* administration of the material, and "excretability, which is a property of being excreted outside the body without accumulation after in vivo administration of the material." Thus, the term "biocompatible dispersion stabilizer" refers to a component that improves the dispersibility of particles while having the biocompatibility described above.

As used herein, the term "biodegradability" refers to a property of the material to naturally decompose when exposed to a physiological solution having a pH of 6 to 8, and specifically, refers to a property in which the material may be decomposed over time by body fluids, decomposing enzymes, or microorganisms, etc., *in vivo.*

According to a specific embodiment of the invention, the cerium oxide nanoparticles used in the present invention may be selected from the group consisting of cerium (IV) oxide (CeO₂) nanoparticles, cerium (III) oxide (Ce₂O₃) nanoparticles, or a mixture thereof.

According to a specific embodiment of the invention, in Formula 1, R₁ is hydrogen, R₂ is oxygen, and l is 1. According to an octet rule, it is obvious that when R₁ is hydrogen, - - - is a single bond, and when R₂ is oxygen, - - - is a double bond. The compound of Formula 1 wherein R₁ is hydrogen, R₂ is oxygen, and l is 1 is polyvinylpyrrolidone (PVP).

According to a specific embodiment of the invention, the polymer represented by formula 1 and the cerium oxide nanoparticles have a content ratio of 7:1 to 11:1.

The present inventors observed that when the polymer of formula 1 and the cerium oxide nanoparticles within the nanocomplex of the present invention have a content ratio of 7:1 to 11:1 (7-11 mg PVP/mg Ce), they exhibit optimal biostability, dispersibility, and maximized anti-inflammatory effect, thereby possessing the most outstanding particle characteristics. This confirms that, compared to conventional cerium oxide nanocomplex requiring additional coating of the outermost layer with dispersing stabilizers such as PGA (polyglutamic acid), the present invention significantly enhances biocompatibility and therapeutic efficacy while dramatically improving the efficiency and economy of mass production through structural simplification.

More specifically, the polymer represented by formula 1 and the cerium oxide nanoparticles have a content ratio of 7.5:1 to 10.9:1, more specifically 8:1 to 9.5:1, even more specifically 8.5:1 to 9:1, and most specifically, have a content ratio of approximately 8.7:1.

According to a specific embodiment, the nanocomplex of the present invention further comprises a multifunctional ligand represented by the following Formula 2:

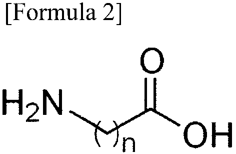

wherein n is an integer of 3 to 7.

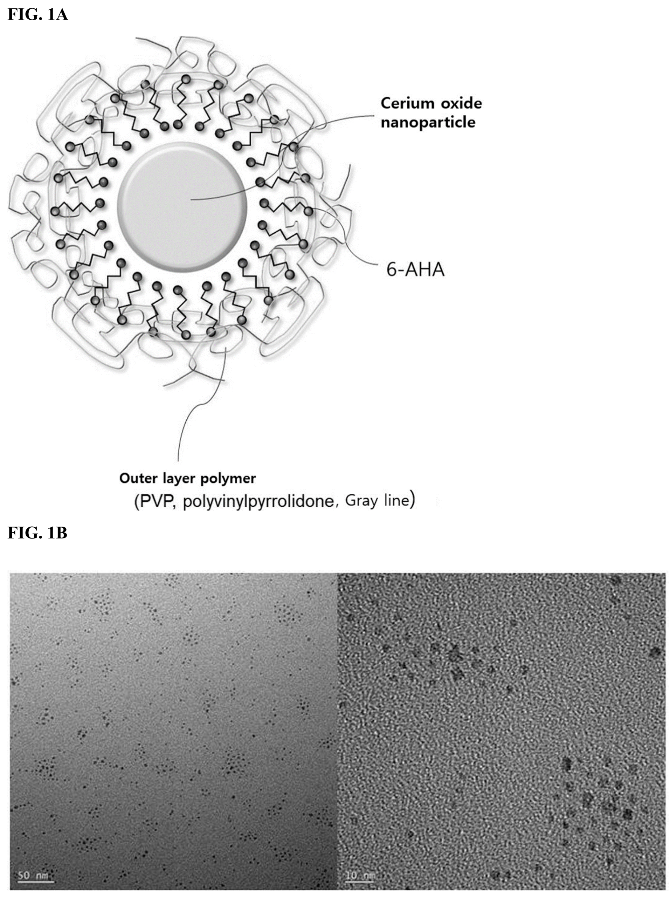

As used herein, the term "multi-functional ligand" refers to a molecule having two or more active functional groups and binding to two or more molecules, thereby serving as a linker between the molecules. The multi-functional ligand of Formula 2 used in the present disclosure has a carboxyl group capable of binding to cerium oxide nanoparticles and an amine group capable of binding to the compound of Formula 1 (e.g., PVP) in the outer layer, enabling the nanocomplex of the present invention to be formed more efficiently and stably. Therefore, the multi-functional ligand of Formula 2 may exist within the outer layer or between the core layer and the outer layer. In this case, the carboxyl group of the multi-functional ligand may be oriented toward the core, and the amino group may be oriented toward the compound of Formula 1, exhibiting directional alignment (FIG. 1A).

According to a specific embodiment, in the above Formula 2, n is 5. The compound of Formula 2 wherein n is 5 is 6-aminohexanoic acid (6-AHA).

According to a specific embodiment, the nanocomplex of the present disclosure has an average particle size of 5 nm to 80 nm. More specifically, the nanocomplex has an average particle size of 10 nm to 50 nm, more specifically 15 nm to 30 nm, and most specifically, approximately 20 nm.

According to a specific embodiment of the invention, the nanocomplex of the present disclosure has a surface charge (Zeta potential) of -1.0 to 1.0 mV. More specifically, the nanocomplex has a surface charge (Zeta potential) of -0.5 to 0.5 mV. According to the present disclosure, the nanocomplex exhibits a significantly low surface charge converging to 0 mV, thereby minimizing interaction with the aqueous *in vivo* environment upon human injection and demonstrating excellent biocompatibility.

In another aspect of this invention, there is provided a composition for preventing or treating inflammatory or autoimmune diseases, comprising the aforementioned nanocomplex of the present invention as an active ingredient.

In still another aspect of this invention, there is provided a method for preventing or treating inflammatory or autoimmune diseases, comprising administering the aforementioned nanocomplex of the present invention to a subject in need thereof.

The cerium oxide nanocomplex used in the present disclosure has already been described in detail above, and thus description thereof is omitted to avoid excessive overlap.

As used herein, the term "prevention" refers to inhibiting the development of a disease or condition in a subject who has not been diagnosed with the disease or condition, but is likely to be afflicted with such a disease or illness.

As used herein, the term "treatment" refers to (a) inhibiting the progression of the disease, illness, or symptom; (b) alleviating the disease, illness, or symptom; or (c) eliminating the disease, illness, or symptom. The composition according to the present disclosure reduces reactive oxygen species to inhibit, eliminate or alleviate the development of symptoms caused by an excessive or unwanted immune response or inflammation. Specifically, as seen in the following examples, the composition of the present invention significantly improves the survival rate of subjects with severe cerebral infarction and substantially reduces the volume of the infarct lesion. Therefore, the composition according to the present disclosure may itself be a composition for treating inflammatory or autoimmune diseases, specifically cerebral infarction, or may be administered in combination with other pharmacological ingredients having an anti-inflammatory effect and applied as a therapeutic adjuvant for the above diseases. Accordingly, as used herein, the term "treatment" or "therapeutic agent" includes the meaning of "therapeutic aid" or a "therapeutic adjuvant."

As used herein, the term "administration" refers to administration of a therapeutically effective amount of the composition according to the present disclosure directly to a subject so that the same amount is formed in the body of the subject, and has the same meaning as "transplantation" or "injection."

As used herein, the term "therapeutically effective amount" refers to the amount of the composition contained in an amount sufficient to provide a therapeutic or prophylactic effect to an individual who wishes to administer the composition according to the present disclosure, and is therefore meant to include "prophylactically effective amount."

As used herein, the term "subject" includes, without limitation, human, mouse, rat, guinea pig, dog, cat, horse, cow, pig, monkey, chimpanzee, baboon, or rhesus monkey. Specifically, the subject of the present invention is a human.

According to a specific embodiment of the invention, the inflammatory or autoimmune diseases are one or more diseases selected from the group consisting of intracerebral hemorrhage (ICH), cerebral infarction, dermatitis, traumatic brain injury, inflammatory bowel disease (IBD), subarachnoid hemorrhage (SAH), cerebral edema, hypoxic ischemic encephalopathy, stroke, traumatic spinal cord injury, gunshot injury, acute respiratory distress syndrome, cytokine storm syndrome, sepsis, systemic inflammatory response syndrome, peritonitis, multiple sclerosis, rheumatoid arthritis, reactive arthritis, type 1 diabetes, systemic lupus erythematosus, idiopathic pulmonary fibrosis, polymyositis, dermatomyositis, localized scleroderma, systemic scleroderma, Sjogren's syndrome, Raynaud's phenomenon, Bechet's disease, Kawasaki's disease, primary biliary sclerosis, primary sclerosing cholangitis, psoriasis, myasthenia gravis, autoimmune vasculitis, primary angiitis of the central nervous system, and inflammatory liver disease.

According to a specific embodiment, the inflammatory or autoimmune disease is intracerebral hemorrhage (ICH).

As used herein, the term "intracerebral hemorrhage (ICH)", also referred to as "intracranial hemorrhage", refers to a disease where brain tissue is contaminated and damaged by blood leaked from damaged cerebral blood vessels. Physical damage in brain tissue occurs due to the volume of the hematoma primarily generated by blood leakage from cerebral blood vessels. Subsequently, an inflammatory response is induced by cytotoxic substances such as reactive oxygen species, heme, and thrombin, leading to edema around the hematoma. Therefore, to suppress the symptoms of intracerebral hemorrhage, it is crucial to rapidly inhibit the edema and inflammatory response around the hematoma to prevent resulting neuronal damage at an early stage.

According to a specific embodiment, the inflammatory or autoimmune disease is cerebral infarction.

As used herein, the term "cerebral infarction" refers to a disease where ischemic damage occurs due to occlusion of cerebral blood vessels, leading to neurological dysfunction. Ischemic brain damage caused by cerebral infarction, similar to hematoma formation caused by intracerebral hemorrhage described above, leads to excessive inflammatory responses and ultimately becomes one of the primary causes of cerebral edema. Therefore, "cerebral edema" may also be included among the inflammatory or autoimmune diseases preventable or treatable with the composition of the present invention.

As used herein, the term "cerebral edema" refers to a condition where brain tissue becomes enlarged due to the influx of fluid into cells or tissues caused by any damage to brain tissue, such as trauma, tumors, or inflammation. Cerebral edema arises as a result of the inflammatory response within brain tissue. When extensive cerebral edema occurs within the confined cranial cavity, it exerts physical pressure on surrounding tissues. In severe cases, this can lead to brain herniation, brainstem compression and hydrocephalus, and potentially causing additional neurological damage or even death.

According to specific embodiments of the present invention, the cerebral edema to be prevented or treated by the present invention includes that caused by stroke, cerebral infarction, intracranial hemorrhage, neoplasm of the brain, traumatic brain injury, encephalitis, inflammatory brain disease, demyelinating brain disease, or intracranial vasculitis. More specifically, the brain edema is that caused by cerebral infarction.

According to a specific embodiment, the inflammatory or autoimmune disease is dermatitis.

As used herein, the term "dermatitis" refers to any disease in which skin tissue including the epidermis, dermis, and subcutaneous tissue, is damaged and the inherent biological functions of the skin tissue, including its barrier function, are impaired due to an excessive or unwanted immune response or the inflammation induced thereby. Dermatitis preventable or treatable by the compositions of the present invention may be selected from the group consisting of atopic dermatitis, eczema, erythema multiforme, erythema nodosum and pyoderma gangrenosum, and most specifically may be atopic dermatitis.

As used herein, the term "atopic dermatitis" refers to a chronic allergic inflammatory skin disease that commonly occurs in children and can persist into adulthood. Atopic dermatitis is influenced by a family history of allergic diseases and is triggered by allergens such as food or house dust mites. Most currently used medications, including steroid preparations, remain largely symptomatic treatments and fail to achieve fundamental elimination of the underlying cause, leaving it a representative intractable skin disease.

According to a specific embodiment, the inflammatory or autoimmune disease is traumatic brain injury.

As used herein, the term "traumatic brain injury" refers to a condition where brain tissue is damaged by an external physical force, leading to impaired brain functions, including cognitive abilities. Oxidative stress caused by excessive generation of reactive oxygen species (ROS) post-trauma is a major secondary damage mechanism in traumatic brain injury. Reducing the explosive generation of ROS immediately after trauma is critically important for preventing further tissue damage. Furthermore, similar to the formation of hematomas due to intracerebral hemorrhage and ischemic brain damage due to cerebral infarction described above, traumatic brain injury is also one of the major causes ultimately leading to cerebral edema. Therefore, the prevention or treatment of traumatic brain injury using the composition of the present invention can lead to the prevention or treatment of "cerebral edema".

According to a specific embodiment, the inflammatory or autoimmune disease is gunshot injury.

As used herein, the term "gunshot injury (or gunshot wound)" refers to damage caused by a projectile, such as a bullet fired from a firearm, being inserted into or penetrating bodily tissue. It is used synonymously with "gunshot-induced inflammation" or "gunshot-induced sepsis" in the present disclosure. Damage caused by gunshot wounds includes bleeding, fractures, organ damage, wound infection, inflammation, and death. Long-term complications include intestinal obstruction, growth impairment, neurogenic bladder and paralysis, recurrent respiratory distress and pneumothorax, and early dementia due to hypoxic brain damage. In particular, a penetrating bullet forms a perforation in the tissue, causing bleeding and the entry of foreign matter, leading to inflammation and sepsis. Therefore, controlling the rapid escalation of inflammatory response and reactive oxygen species immediately after a gunshot wound, along with surgical suturing, is a critical factor determining the patient's survival.

According to a specific embodiment, the inflammatory or autoimmune disease is acute respiratory distress syndrome.

As used herein, the term "acute respiratory distress syndrome (ARDS)", also referred to as "acute lung injury (ALI)" refers to a pathological state where respiratory failure is induced by a decrease in arterial blood oxygen partial pressure and accumulation of carbon dioxide due to a sudden decline in lung function. Representative causes of sudden lung dysfunction include sepsis due to systemic infection, trauma, massive blood transfusion, and pancreatitis. Massive influx of foreign substances due to bacterial infection or trauma, causes severe inflammation in the lungs, leading to a sudden loss of lung function. Acute respiratory distress syndrome is classified into acute phase, proliferative phase, and fibrotic phase based on its course. Since the acute phase is primarily driven by excessive inflammatory response, controlling inflammation is paramount to halting disease progression.

According to a specific embodiment, the inflammatory or autoimmune disease is inflammatory bowel disease (IBD).

As used herein, the term "inflammatory bowel disease (IBD)" encompasses inflammatory disorders occurring in gastrointestinal tract tissues, including but not limited to the most common forms: ulcerative colitis and Crohn's disease. Inflammatory bowel disease is a chronic and intractable disease characterized by symptoms such as abdominal pain, diarrhea, bloody stools, and weight loss persisting for months or longer. Fundamental treatment requires controlling the inflammation occurring throughout the digestive tract and the resulting multiple oxidative stresses.

According to a specific embodiment, the inflammatory or autoimmune disease is subarachnoid hemorrhage (SAH).

As used herein, the term "subarachnoid hemorrhage (SAH)" refers to a disease where blood leaks from cerebral blood vessels into the subarachnoid space, causing various inflammatory reactions and brain cell damage in the brain tissue located near the brain parenchyma. Being a hemorrhage occurring within the subarachnoid space that broadly envelops the brain, SAH causes extensive damage throughout the brain. Consequently, it often leads to immediate death or severe permanent brain damage upon occurrence. The brain damage resulting from subarachnoid hemorrhage is triggered by a cascade of inflammatory responses in the brain tissue initiated by blood-derived components such as hemoglobin and thrombin, which subsequently induce oxidative damage via reactive oxygen species. Therefore, rapid control of reactive oxygen species and prompt suppression of inflammatory responses are critical to minimizing neurological damage.

According to a specific embodiment, the inflammatory or autoimmune disease is sepsis.

As used herein, the term "sepsis" encompasses a harmful systemic inflammatory response to external infectious agents such as viruses or bacteria and the resulting pathological state, including complications like "severe sepsis" and "septic shock". Sepsis is accompanied by fever, respiratory distress, and rapid changes in white blood cell counts, leading to multiple organ failure and ultimately in a high mortality rate. Simultaneous measures are taken such as administering antibiotics and antifungals for elimination of the infectious agent, providing fluid resuscitation, maintaining blood pressure, performing blood transfusions and correcting acidosis for alleviation of septic shock. However, fundamental treatment requires eliminating the excessive reactive oxygen species produced by the inflammatory response.

According to a specific embodiment, the inflammatory or autoimmune disease is inflammatory liver disease.

As used herein, the term "inflammatory liver disease" refers to a series of diseases accompanied by liver tissue damage that arises, directly or indirectly, from excessive or undesired immune responses or inflammatory reactions caused by various etiologies.

More specifically, the inflammatory liver disease is selected from the group consisting of viral hepatitis, toxoplasma hepatitis, alcoholic liver disease, toxic liver disease, acute and subacute hepatic failure, liver abscess, nonspecific reactive hepatitis, liver infarction, hepatic veno-occlusive disease, injury of liver or gallbladder, and liver transplantation-related hepatitis.

When the composition according to the present disclosure is prepared as a pharmaceutical composition, the pharmaceutical composition according to the present disclosure contains a pharmaceutically acceptable carrier. Pharmaceutically acceptable carriers contained in the pharmaceutical composition according to the present disclosure are commonly used in preparation, and examples thereof include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil, etc. The pharmaceutical composition according to the present disclosure may further contain lubricants, wetting agents, sweetening agents, flavoring agents, emulsifying agents, suspending agents, preservatives, etc., in addition to the above components. Suitable pharmaceutically acceptable carriers and formulations are described in detail in Remington's Pharmaceutical Sciences (19th ed., 1995).

The pharmaceutical composition according to the present disclosure may be administered through various routes of administration, specifically, parenterally, and more specifically, intravenously, intraarterially, subcutaneously, intraperitoneally, intradermally, intramuscularly, intraventricularly, intrathecally, inhalationally, nasally, intraarticularly or topically.

A suitable dosage of the pharmaceutical composition according to the present disclosure may be variously prescribed depending on factors such as formulation method, administration mode, age, weight, sex, pathological condition, food, administration time, administration route, excretion rate, and response sensitivity of the patient. A preferred dosage of the pharmaceutical composition according to the present disclosure is within the range of 0.0001 to 1000 mg/kg for adults.

The pharmaceutical composition according to the present disclosure may be manufactured in unit dosage form or filled into a multi-dose container by formulation using pharmaceutically acceptable carriers and/or excipients, according to methods readily practicable by one skilled in the art to which the invention pertains. The dosage form may be a solution, suspension, syrup, or emulsion in an oil or aqueous medium, or may be an extract, powder, granule, tablet, or capsule form, and may additionally contain a dispersant or stabilizer.

In still another aspect of this invention, there is provided a method for preventing or treating inflammatory or autoimmune diseases, comprising administering to a subject in need thereof a cerium oxide nanocomplex according to the present disclosure or a pharmaceutical composition containing the same as an active ingredient. The cerium oxide nanocomplex used in the present disclosure and the inflammatory or autoimmune diseases that may be prevented or treated by the cerium oxide nanocomplex have already been described in detail above, and thus the description thereof is omitted to avoid excessive overlap.

In still another aspect of this invention, there is provided a method for preparing a cerium oxide nanocomplex, the method comprising:
(a) preparing a mixed solution by adding a cerium precursor, a polymer represented by the following Formula 1, and a crosslinking compound represented by the following Formula 2 to a C₁-C₃ alcoholic solvent; 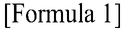 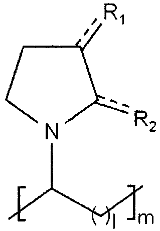 wherein R₁ and R₂ are each independently hydrogen or oxygen, - - - represents a single bond or a double bond, l is 1 or 2, and m is an integer of 100 to 1000;

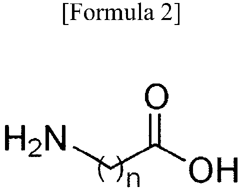

   wherein n is an integer of 3 to 7;
(b) sequentially heating and cooling the mixed solution to obtain cerium oxide nanoparticles; and
(c) washing the cerium oxide nanoparticles with an inorganic salt solution.

According to the present invention, performing step (a) allows the formation of a core-shell structure wherein a compound of Formula 1 is bonded to the cerium oxide nanoparticle core via a crosslinking compound of Formula 2, thereby coated outer layer is formed.

As used herein, the term "coating" refers to forming a new layer having a certain thickness by applying a specific material on a target surface. The target surface and the coating material may be bonded through ionic or non-covalent bond. The term "non-covalent bond" encompasses physical bonds such as adsorption, cohesion, entanglement, and entrapment, as well as bonds arising from the interaction such as hydrogen bonds and van der Waals bonds, acting either alone or in conjunction with the physical bonds described above. When the compound of Formula 1 is coated onto the surface of cerium oxide nanoparticles, it may form a completely enclosed layer surrounding the coated surface or a partially enclosed layer.

According to a specific embodiment of the invention, the C₁-C₃ alcohol solvent is ethanol.

According to a specific embodiment of the invention, the cerium precursor is one or more precursors selected from the group consisting of cerium (III) acetate hydrate, cerium (III) acetylacetonate hydrate, cerium (III) carbonate hydrate, cerium (III) fluoride, cerium (III) chloride, cerium (III) chloride heptahydrate, cerium (III) bromide, cerium (III) iodide, cerium (III) nitrate hexahydrate, cerium (III) oxalate hydrate, cerium (III) sulfate, and cerium (III) sulfate hydrate. More specifically, the cerium precursor is cerium (III) nitrate hexahydrate.

According to a specific embodiment of the invention, the heating is performed at 60 to 80°C. More specifically, it is performed at 65 to 75°C, and most specifically, it is performed at approximately 70°C.

According to a specific embodiment of the invention, the cooling is performed by lowering the temperature to 35 to 50°C. More specifically, it is performed by cooling to 40 to 50°C, and most specifically, by cooling to approximately 45°C.

As used herein, the term "washing" refers to the process of physically and chemically removing impurities bound to the reaction product using a washing solution or buffer. Washing is performed during the separation and purification of the reaction product to reduce the content of undesirable impurities and increase the purity of the desired final product.

As used herein, the term "inorganic salt" refers to a salt derived from inorganic materials that does not contain C-H bonds, wherein cations and anions are combined through ionic bonding in an aqueous solution. According to a specific embodiment, the inorganic salt is selected from the group consisting of NaCl, CaCl₂, KCl, MgCl₂ and combinations thereof.

More specifically, the inorganic salt solution is NaCl solution.

According to a specific embodiment, the inorganic salt solution has a concentration of 0.15 to 0.35 M.

The present inventors discovered that simply adding a step of washing with a 0.15 to 0.35 M sodium chloride solution after reaction completion allows efficient acquisition of particles with an optimal uniform diameter while completely eliminating toxicity from unreacted residues and nitrates. More specifically, the NaCl solution of the present invention has a concentration of 0.20 to 0.30 M, and most specifically, has a concentration of approximately 0.25 M.

### Advantageous Effects

The features and advantages of the present invention are summarized as follows:
(a) The present invention provides a cerium oxide nanocomplex, a method for preparing the same, and a composition for preventing or treating inflammatory or autoimmune diseases comprising the cerium oxide nanocomplex as an active ingredient.
(b) The present invention may be used as an excellent therapeutic composition where both biostability and *in vivo* reactive oxygen species-scavenging efficiency are maximized by modifying the surface of cerium oxide nanoparticles with a pyrrolidone-derived polymer at an optimal content.
(c) The method for preparing the nanocomplex of the present invention can produce uniform particles having an optimal diameter while completely removing reaction residues and nitrate-derived toxicity through a simple process of washing with a sodium chloride solution of an indicated concentration.
(d) The nanocomplex of the present invention significantly suppresses inflammatory responses in various tissues. It is particularly can be advantageously applied as an excellent therapeutic composition that minimizes neuronal damage caused by excessive inflammatory responses in the peri-hematomal region following intracerebral hemorrhage, thereby restoring neurological function and significantly improving patient survival.

### Brief Description of Drawings

FIG. 1 shows a schematic illustration (Fig. 1A) and a transmission electron microscopy (TEM) observation image (Fig. 1B) of the cerium oxide nanocomplex of the present invention.
FIG. 2 shows the particle size after synthesis, dispersion stability in a biomimetic environment, and changes in surface charge according to the content of the modified polymer PVP (polyvinylpyrrolidone) on the outer surface of the cerium oxide nanocomplex of the present invention (FIG. 2A) and the amount of PVP contained in the dried cerium oxide nanocomplex of the present invention, expressed per unit cerium (FIG. 2B).
FIG. 3 shows the particle size of the cerium oxide nanocomplex of the present invention over time when exposed to a biomimetic environment measured by a dynamic light scattering device.
FIG. 4 shows the comparison of surface charges between the conventionally developed cerium oxide nanocomplex (CX213) and the cerium oxide nanocomplex of the present invention.
FIG. 5 shows the reaction residue (FIG. 5A) and particle size changes (FIG. 5B) according to the concentration of the sodium chloride aqueous solution used in the purification the cerium oxide nanocomplex.
FIG. 6 shows the comparative analysis of the therapeutic effects of the conventionally developed cerium oxide nanocomplex (CX213) and the cerium oxide nanocomplex of the present invention (CX301) on severe cerebral infarction, evaluated by survival rates after intravenous injection in a severe cerebral infarction (MIS) rat model.
FIG. 7 shows the results of a comparative analysis of the therapeutic effects of the conventionally developed cerium oxide nanocomplex (CX213) and the cerium oxide nanocomplex (CX301) of the present invention on severe cerebral infarction, based on infarct volume.
FIG. 8 shows the results of comparing and analyzing brain water content due to cerebral edema to evaluate the therapeutic effect of the cerium oxide nanocomplex of the present invention on intracerebral hemorrhage.
FIG. 9 shows the cylinder symmetric score in a traumatic brain injury model to evaluate the therapeutic effect of the cerium oxide nanocomplex of the present invention on traumatic brain injury.
FIG. 10 illustrates the evaluation of the therapeutic efficacy of the cerium oxide nanocomplex of the present invention in a 2,4-dinitro-chlorobenzene (DNCB)-induced atopic dermatitis model, showing the extent of transepidermal water loss (FIG. 10A) and the degree of epidermal thickening and infiltration of inflammatory mast cells (FIG. 10B), respectively.
FIG. 11 shows the results of measuring body weight changes and the disease activity score (DAI) in a dextran sodium sulfate (DSS)-induced inflammatory bowel disease animal model to evaluate the therapeutic effect of the cerium oxide nanocomplex of the present invention on inflammatory bowel disease.
FIG. 12 shows the results of measuring changes in survival rate following treatment with the cerium oxide nanocomplex of the present invention in a CLP-induced sepsis animal model to evaluate its therapeutic effect on sepsis.
FIG. 13 shows the results of measuring cell survival rates by treating hepatocytes (hepa-1c1c7) induced with toxic stimulation by pyrogallol, with the cerium oxide nanocomplex of the present invention to confirm the therapeutic effect on inflammatory liver disease.
FIG. 14 shows the results of measuring changes in survival rates following treatment with the cerium oxide nanocomplex of the present invention in an animal model with induced subarachnoid hemorrhage, to evaluate the therapeutic effect on subarachnoid hemorrhage.

### Mode for Invention

Hereinafter, the present invention will be described in more detail by way of examples. These examples are only for illustrating the present invention in more detail, and it will be apparent to those skilled in the art that the scope of the present invention according to the subject matter of the present invention is not limited by these examples.

### Examples

### Example 1: Synthesis of Cerium Oxide Nanocomplex

A first solution was prepared by dissolving 6-aminohexanoic acid (37.75 g, Sigma-Aldrich, St. Louis, MO) in deionized water (1.7 L). Ethyl alcohol (1.4 L) was added to the first solution while stirring, followed by the addition of 132.83 g of polyvinylpyrrolidone (PVP, Ashland). The mixture was then heated to 70°C in air to prepare a second solution. Meanwhile, a third solution was prepared by dissolving cerium (III) nitrate hexahydrate (Ce(NO₃)₃·6H₂O, 31.05 g, Alfa Aesar, Ward Hill, MA) in ethyl alcohol (2.9 L) at room temperature (approximately 20°C). Subsequently, the third solution was added to the second solution to prepare a fourth solution. The temperature of the fourth solution was maintained at 70°C for 2 hours, then gradually lowered to 45°C. Through this process, cerium oxide nanoparticles with 6-aminohexanoic acid and polyvinylpyrrolidone bound to their surfaces were obtained (FIGS. 1a and 1b). Subsequently, the nanoparticles were washed five times with a mixture of acetone and sodium chloride aqueous solution to remove unreacted materials.

### Example 2: Search for the Optimal Content of Polyvinylpyrrolidone Used as the Outer Layer Polymer

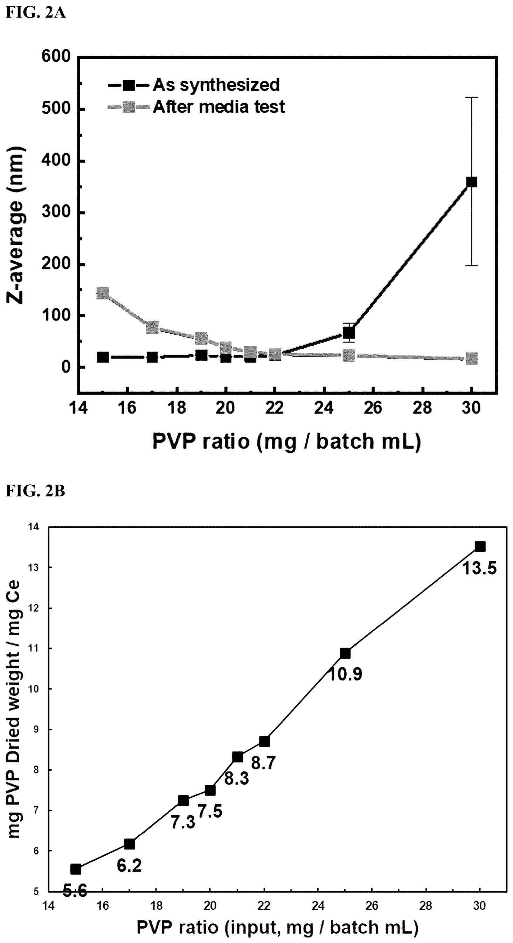

To determine the optimal content of the polyvinylpyrrolidone (PVP) dispersion stabilizer forming the outer layer of the cerium oxide nanoparticles, the particle formation tendency and dispersion stability in a biomimetic environment were analyzed as a function of PVP content. During the synthesis of the cerium oxide nanocomplex in Example 1, the PVP content ratio was adjusted from 15 to 30 mg PVP/total batch mL. The particle size of the synthesized product and its dispersion stability (Z-Average, nm) and surface charge (Zeta-potential, mV) in a biomimetic environment containing protein and sodium bicarbonate. The results showed that the particle characteristics were optimized when PVP was applied at a content ratio of 20-25 mg PVP/total batch mL (FIG. 2A).

Furthermore, to more clearly measure the derived optimal PVP content range based on the final product, i.e. the nanocomplex, the entire synthesized nanocomplex was freeze-dried and then the PVP content within the dried nanocomposite was quantified and converted to the amount per unit cerium. As a result, it was found that the final nanocomplex exhibited the most favorable particle characteristics when the content ratio of PVP to the cerium oxide nanoparticles was in the range of 7.5 to 10.9 (7.5 - 10.9 mg PVP/mg Ce), with the optimal ratio identified as 8.7 (FIG. 2B). This composition ratio explored in the present invention can be considered the result of identifying the most suitable conditions, considering the impact of nanocomplex size on dispersion, functionality, and biological stability.

### Experimental Example 1: Comparison of Biostability and Dispersibility Between Conventionally Developed Particles and Particles of the Present Invention in a Biomimetic Environment

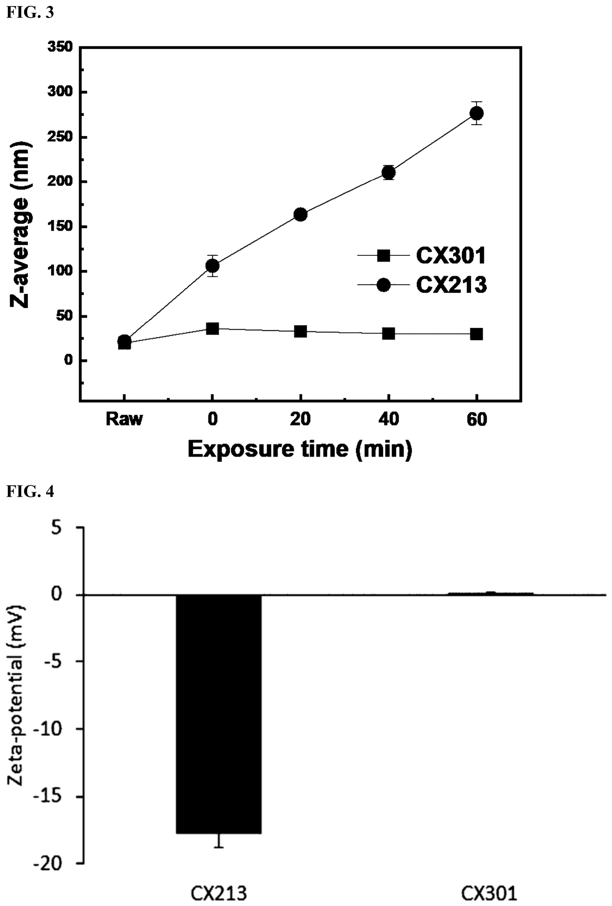

The biostability (dispersibility) in a biomimetic environment was compared between CX213, a ceria nanocomplex previously developed by the present inventors, in which polyglutamic acid (PGA) is introduced as an additional dispersion-stabilizing outermost layer, and CX301, the cerium oxide nanoparticles of the present invention where such an outer polymer layer is omitted. For this, particle size changes over time in 0.05M PBS (phosphate-buffered saline) was analyzed. Samples were collected at 0, 20, 40, and 60 minutes, and particle size was analyzed using dynamic light scattering equipment. The results showed that as reaction time progressed, the CX213 nanocomplex exhibited particle aggregation reaching hundreds of nm, whereas the CX301 of the present invention maintained a constant particle size over time (FIG. 3).

### Experimental Example 2: Comparison of Surface Charge Between Conventionally Developed Particles and Particles of the Present Invention

The inventors measured the surface charge of the conventionally developed CX213 and the CX301 of the present invention to evaluate differences in biostability and dispersibility based on the presence or absence of PVP optimization. Dispersing each synthesized cerium oxide nanocomplex in deionized water and analyzing the surface characteristics of the cerium nanocomplex using a dynamic light scattering instrument revealed a surface charge of -17.5 mV for CX213 and 0 mV for CX301, showing a clear difference between the two particles (FIG. 4). This indicates that the present invention, converging to a surface charge of 0 mV, minimizes interactions in the *in vivo* environment compared to CX213 possessing a strong negative charge due to terminal PGA polymers.

### Experimental Example 3: Evaluation of the Effect of Sodium Chloride Solution During Purification on Reaction Residue Concentration and Particle Size

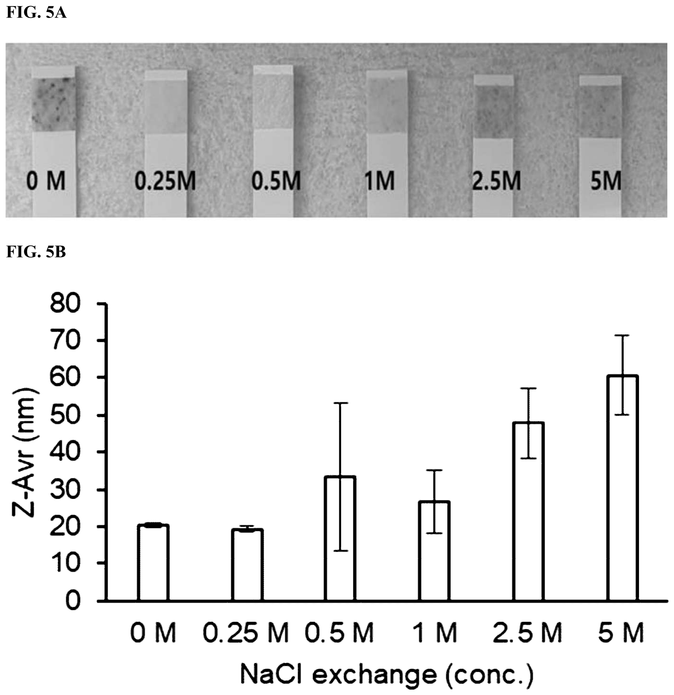

To measure the reaction residues and the particle size of final products of cerium oxide nanocomplex obtained by adding sodium chloride solutions of different concentrations during purification, the concentration of the sodium chloride aqueous solution was adjusted to 0, 0.25, 0.5, 1.0, 2.5, and 5 M during the preparation of the cerium oxide nanocomplex in Example 1. The resulting reaction residues, particularly the residual nitrate content, were measured using a nitrate indicator pad (PP strip, Johnson). Additionally, particle size was confirmed using dynamic light scattering equipment. As shown in FIG. 5A, using a 0.25 M sodium chloride solution during the purification process was most effective at removing reaction residues, particularly nitrate. This demonstrated that the manufacturing process of the present invention is efficient for both reducing residues in pharmaceuticals and eliminating toxicity caused by nitrate. Furthermore, particle size was also optimized (20 nm) when using the 0.25 M sodium chloride solution, confirming that addition of sodium chloride significantly affects particle formation stability (FIG. 5B).

### Experimental Example 4: Evaluation of Therapeutic Effect on Non-infectious Inflammatory Diseases by Survival Rate

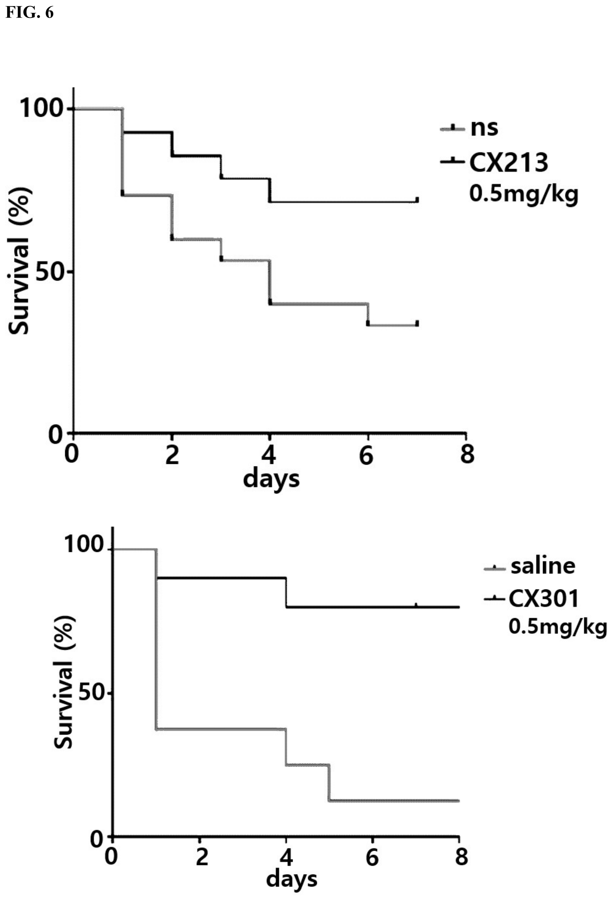

To comparatively evaluate the therapeutic effects on non-infectious inflammatory diseases between the conventionally developed cerium oxide nanocomplex CX213 and the CX301 of the present invention prepared in Example 1, SD (Sparague-Dawley) rats (Coatech Co., Ltd.) were anesthetized with isoflurane. The left middle cerebral artery was ligated with 4-0 proline suture to induce severe cerebral infarction (MIS), a representative non-infectious inflammatory disease. One hour after inducing severe cerebral infarction, each of the two types of cerium oxide nanocomplex was intravenously injected at a dose of 0.5 mg Ce/kg over 5 minutes. As a control group, an equal volume of saline was injected. The survival rate was measured by periodically checking the mortality status of SD rats from 7 days after inducing MIS. Experiments were conducted on 18 SD rats of experimental group and 15 SD rats in the control group, and the average value was expressed as the survival rate. As shown in FIG. 6, the CX213 nanocomplex demonstrated a survival rate increase of 2.25 times compared to the control group (control group: 28.57%, CX213 treated group: 64.28%), while the group injected with CX301 of the present invention showed a survival rate increase of up to 6 times (control group: 13.33%, CX301 administered group: 80.00%).

This confirmed that the novel cerium oxide nanocomplex CX301 of the present invention can be effectively utilized as a therapeutic composition for non-infectious inflammatory diseases, as it was observed to possess a therapeutic effect 2.6 times greater than that of CX213, previously developed by the present inventors.

### Experimental Example 5: Evaluation of Therapeutic Effect on Non-infectious Inflammatory Diseases by Infarct Volume

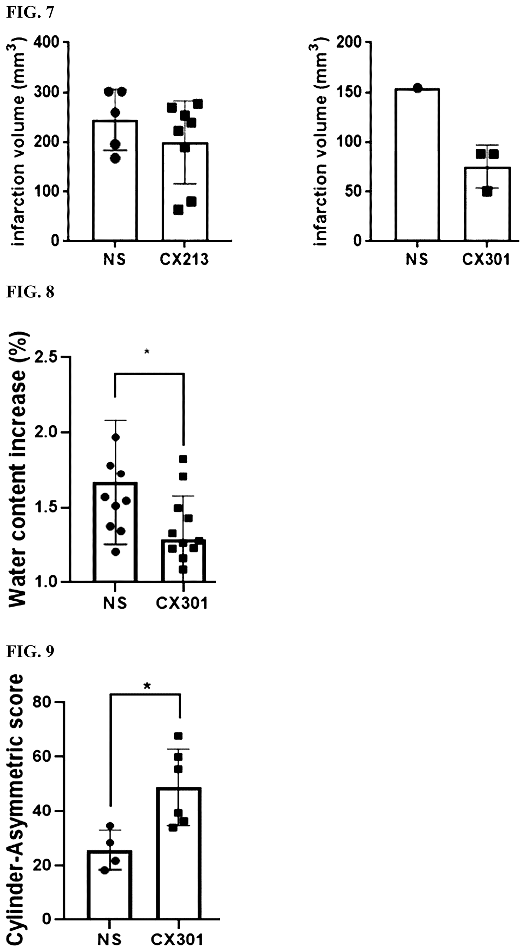

Brain tissue was harvested from SD rats in the Experimental Example 4 to obtain coronal sections, and Nissl staining was performed to confirm the degree of infarction. After staining the tissue, the volume was calculated based on the area of each section. Compared to the control group, the CX213-treated group showed a 18.58% reduction in infarct volume, while the CX301 group showed a 51.22% reduction in infarct volume. Thus, the cerium oxide nanocomplex of the present invention demonstrated a therapeutic effect 2.76 times greater than that of previously developed nanocomplex, when evaluated based on infarct volume (FIG. 7). This demonstrated that the CX301 nanocomplex of the present invention not only significantly increased survival rates but also substantially reduced infarct volume in actual brain tissue, confirming its markedly superior therapeutic effect from multiple perspectives.

### Experimental Example 6: Evaluation of Therapeutic Effect on Additional Non-infectious Inflammatory Diseases

### Intracerebral Hemorrhage

Intracerebral hemorrhage is a disease characterized by persistent neuronal damage due to excessive inflammatory responses around the hematoma. The present inventors sought to confirm whether the anti-inflammatory action of the cerium oxide nanocomplex of the present invention could alleviate symptoms of intracerebral hemorrhage. First, randomly assigned SD rats were anesthetized with 2.5% isoflurane. The rat's head was fixed in a stereotaxic frame, and a cranial burr hole was created at the following coordinates relative to bregma: 0.02 mm posterior to the coronal plane and 0.30 mm lateral to the midline. To induce intracerebral hemorrhage, 1µl of collagenase VII (0.5 units) was injected to a depth of 0.66 mm. Subsequently, either the cerium oxide nanocomplex (0.5 mg/kg) or saline was administered intravenously twice: 1 hour and 24 hours after model induction. To assess disease severity, brain edema was evaluated on day 3 by measuring brain water content. The brain water content measurement revealed a 22.72% improvement in the cerium oxide nanocomplex-treated group compared to the saline-treated control group (FIG. 8). These results confirm that the cerium oxide nanocomplex of the present invention also exhibits excellent therapeutic effects for intracerebral hemorrhage.

### Traumatic Brain Injury

Traumatic brain injury (TBI) is a disease characterized by secondary neuronal damage resulting from an inflammatory response caused by excessive reactive oxygen species (ROS) production. The present inventors sought to verify whether the cerium oxide nanocomplex of the present invention exhibits therapeutic efficacy for TBI. To induce traumatic brain injury, SD rats were anesthetized with 2.5% isoflurane. The rat's head was fixed in a stereotaxic frame, and a cranial burr hole was created at the following coordinates relative to bregma: 3.5 mm posterior to the coronal plane and 3.5 mm lateral to the midline. To inflict brain injury at the designated site, a 5 mm diameter impactor tip was installed on a pinpoint impactor (PCI3000, Hatteras), set to an impact depth of 6 mm, and an impact was delivered at a velocity of 4 m/s. One hour after inducing the disease, a single intravenous dose of 0.1 mg/kg of the cerium oxide nanocomplex of the present invention was administered, and the degree of motor dysfunction was assessed using the cylinder test. To ensure accurate measurement, a mirror was placed opposite the behavioral recording area to eliminate blind spots. The animal was then placed in the cylinder, and its movements were observed for 10 minutes. Measurements were recorded after confirming the use of the wall for support, balance maintenance, and the foot used for landing. Scores were evaluated using the formula [(I+1/2B)/(I+C+B)]x100, where I represents the ipsilateral (affected) side, C represents the contralateral (unaffected) side, and B represents the bilateral (both sides). Test results showed a 47.02% improvement in motor ability in the cerium oxide nanocomplex group compared to the saline group on day 3 of measurement (FIG. 9). These results confirm that the cerium oxide nanocomplex of the present invention can be effectively applied as a therapeutic composition for the intractable disease of traumatic brain injury.

### Dermatitis

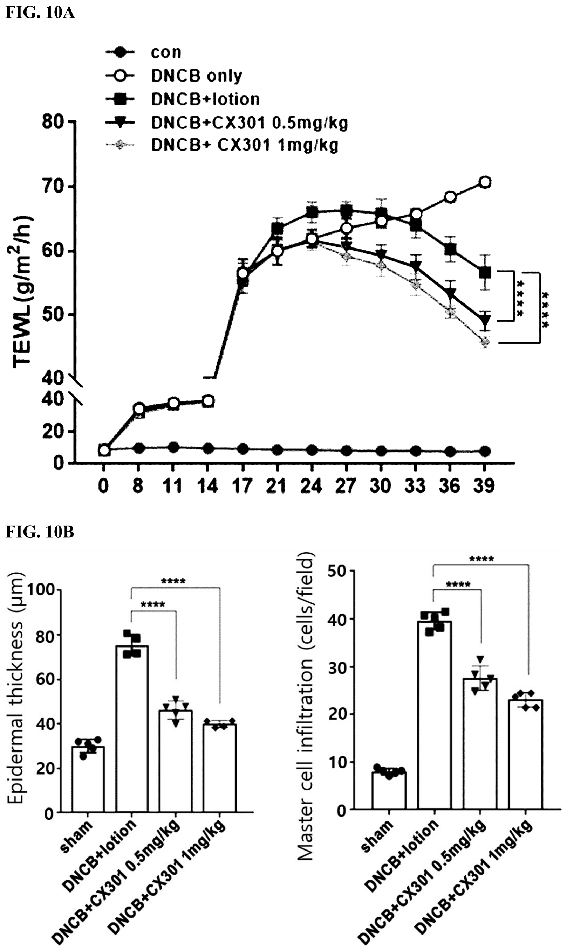

In inflammatory skin diseases caused by various factors, excessive inflammatory responses induced by reactive oxygen species exacerbate the severity of dermatitis. To determine whether the cerium oxide nanocomplex of the present invention can also alleviate the severity of inflammatory skin diseases, dermatitis model was prepared using hairless SKH-1 mice. To induce atopic dermatitis, 0.2 ml of 1% 2,4-dinitrochlorobenzene (DNCB) was applied daily to the backs of the mice for 35 days. Starting on day 17 of application, 0.5 mg/kg or 1 mg/kg of the nanocomplex of the present invention was applied in 0.2 ml doses to the same site. To observe the improvement on dermatitis, the degree of skin moisture loss was measured every 3 days using a moisture measurement device (GP skin barrier, GP skin). On the final day, skin tissue was obtained, and changes in epidermal thickness and the degree of inflammatory cell infiltration were confirmed by staining. The results showed that, by the final day, the 0.5 mg/kg cerium oxide nanocomplex - treated group exhibited a 12.13% improvement in moisture loss, while the 1 mg/kg treatment group showed a 19.19% improvement (FIG. 10A). As for the epidermal thickness, hypertrophy also reduced by 38.78% and 47.09% in the 0.5 mg/kg and 1 mg/kg cerium oxide nanocomplex treated - groups, respectively (FIG. 10B, left). The degree of inflammatory mast cell infiltration, characteristically observed in the dermatitis model, was reduced by 27.21% and 33.05%, respectively (FIG. 10B, right).

### Colitis

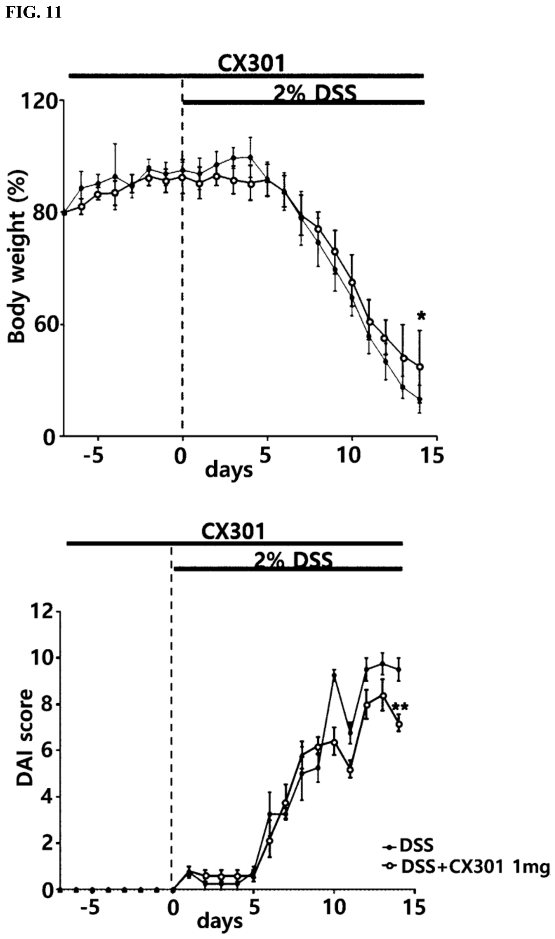

In inflammatory bowel disease (IBD), represented by ulcerative colitis (UC) and Crohn's disease (CD), oxidative stress plays an essential role in pathogenesis and progression. The present inventors sought to verify whether the antioxidant and anti-inflammatory activities of the cerium oxide nanocomplex of the present invention could also suppress the symptoms and progression of inflammatory bowel disease. To establish a disease model, C57BL/6 mice were administered either regular drinking water or 2% DSS (dextran sodium sulfate), an agent inducing epithelial cell damage, daily for 10 days. The cerium oxide nanocomplex was administered rectally at a dose of 1 mg/kg in 0.2 ml daily, starting 7 days before DSS-induced IBD onset and continuing until the end of the test period. To assess disease severity, daily body weight, stool consistency, and rectal bleeding were recorded. Scoring was as follows: 0 points for normal condition; 1 point for weight loss ≤10%; 2 points for weight loss ≤15% with loose stools; 3 points for weight loss within 20%, and 4 points for weight loss exceeding 20% accompanied by diarrhea or bloody stools. This score was used to calculate the Disease Activity Index (DAI) for severity assessment. The results showed that compared to the saline-treated control group, the group administered with the cerium oxide nanocomplex exhibited an 8.05% reduction in weight loss (FIG. 11, left) and a 24.21% reduction in disease severity (FIG. 11, right). This demonstrates that the cerium oxide nanocomplex of the present invention exhibits significant antioxidant and anti-inflammatory effects even under conditions of direct intestinal administration, indicating its potential as an effective therapeutic composition for inflammatory bowel disease.

### Sepsis

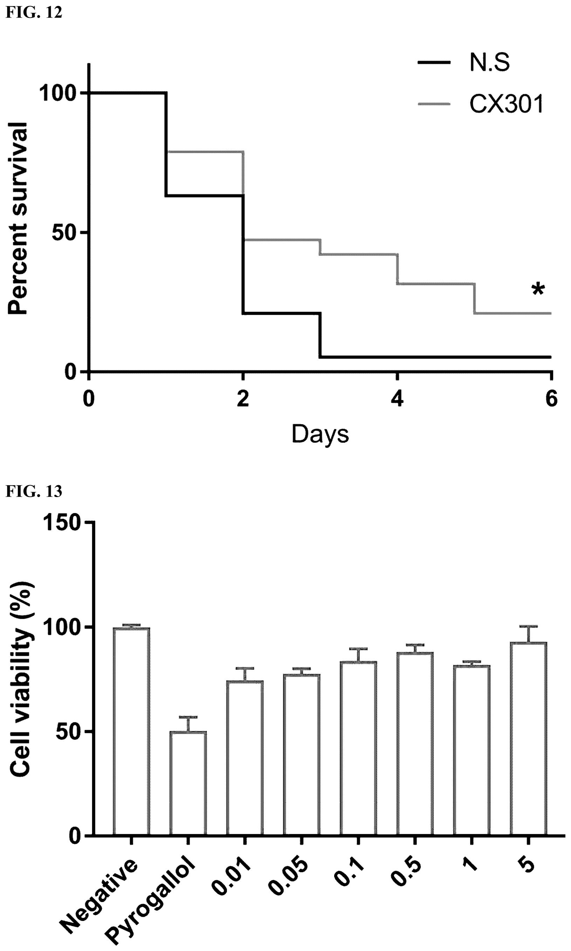

Sepsis is a condition characterized by tissue damage and organ failure resulting from the body's excessive response to various infections. The present inventors sought to verify the therapeutic efficacy of the cerium oxide nanocomplex of the present invention against sepsis. The Cecal Ligation and Puncture (CLP) method was used to induce sepsis. In detail, SD rats were anesthetized with 2.5% isoflurane, then their cecum was ligated and punctured to induce intraperitoneal infection. One hour after disease induction, the cerium oxide nanocomplex of the present invention was administered as a single intravenous dose at a concentration of 0.5 mg/kg based on cerium concentration. Survival rates were measured over 6 days. The experiment was conducted on 19 SD rats each in the experimental and control groups, with survival rates as the outcome measure. The results showed that, compared to the saline-treated control group, the cerium oxide nanocomplex - treated group exhibited approximately a threefold improvement in survival rate by day 6 (control group: 5.3%, experimental group: 15.8%) (FIG. 12). This confirmed that the cerium oxide nanocomplex of the present invention also exhibits excellent therapeutic effects against sepsis.

### Inflammatory liver disease

Inflammatory liver disease is a condition where the liver cannot perform its normal functions due to liver damage, metabolic disorders, and accumulation of toxic substances. The present inventors sought to verify the therapeutic effect of the cerium oxide nanocomplex of the present invention on inflammatory liver disease. To induce the disease, Hepa-1C1C7 hepatocyte cells were treated with 500µM pyrogallol, a cytotoxic substance, to induce acute inflammatory liver disease. Simultaneously, the cells were treated with the cerium oxide nanocomplex of the present invention at concentrations ranging from 0.01 to 5µM. Three hours after treatment, the survival rate of Hepa-lclc7 cells was measured using the NRU assay, a biochemical analysis method that measures cell survival by allowing the neutral red dye to be absorbed by living cells. The results showed that pyrogallol-treated cells exhibited a survival rate of approximately 50.43% compared to the pyrogallol-untreated control group. When treated with the cerium oxide nanocomplex of the present invention, survival rates of 74.55% at a minimum concentration of 0.01 µM and approximately 93.19% at a maximum concentration of 5µM were observed. This represents an increase in survival rate of at least 47.81% to a maximum of 84.78% compared to cells treated with pyrogallol alone (FIG. 13). These results indicate that the cerium oxide nanocomplex of the present invention can also be used as an effective therapeutic composition for inflammatory liver diseases.

### Subarachnoid Hemorrhage

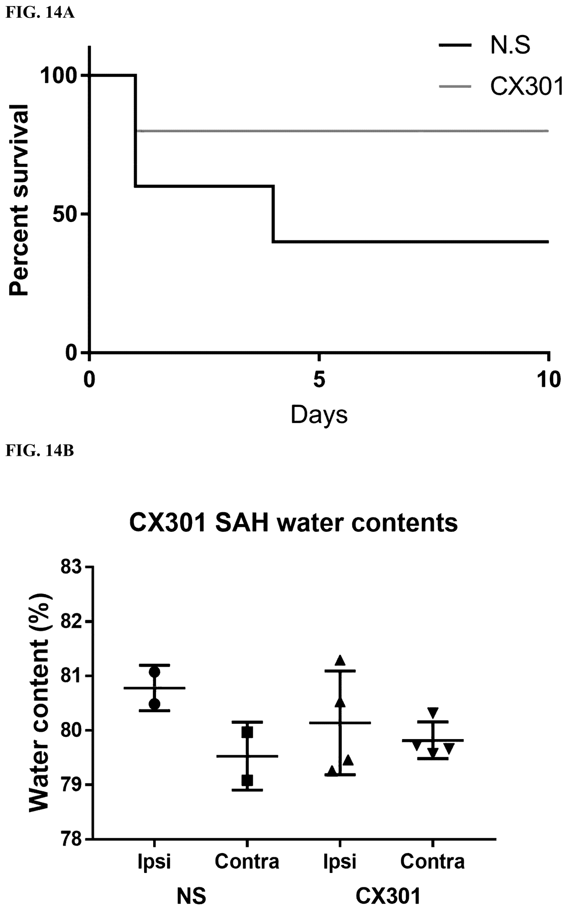

Subarachnoid hemorrhage (SAH) is a condition where blood leaks into the subarachnoid space of the brain, primarily caused by the rupture of a cerebral aneurysm. The present inventors sought to verify the therapeutic efficacy of the cerium oxide nanocomplex of the present invention for subarachnoid hemorrhage. After anesthetizing SD rats with 2.5% isoflurane, the rat's head was fixed, and an incision was made under the neck to locate the common carotid artery, internal carotid artery, and external carotid artery. A filament was inserted into the external carotid artery and advanced to the middle cerebral artery. The middle cerebral artery was then punctured to induce bleeding, causing subarachnoid hemorrhage. One hour after induction, the cerium oxide nanocomplex of the present invention was administered to the rats as a single intravenous dose at a concentration of 0.5 mg/kg based on cerium concentration. Survival rates were measured over 10 days, and recorded for 5 rats each in the control group and the experimental group. The results showed that, compared to the saline-treated control group, the cerium oxide nanocomplex-treated group exhibited a survival rate increase of 2-fold higher by day 10 (control group: 40%, experimental group: 80%) (FIG. 14A). Furthermore, measurement of brain water content on day 3 revealed that while the contralesional side showed a difference of less than 0.3% between the control group (79.5%) and the experimental group (79.8%), the ipsilesional side exhibited a 0.7% reduction in water content in the experimental group (80.8%) compared to the control group (80.1%) (FIG. 14B). This confirmed that the cerium oxide nanocomplex of the present invention also exhibits excellent therapeutic effects for subarachnoid hemorrhage.

Having described specific embodiment of the present invention in detail above, it is to be understood that variants and modifications thereof falling within the spirit of the invention may become apparent to those skilled in this art, and the scope of this invention is to be determined by appended claims and their equivalents.

## Claims

1. A cerium oxide nanocomplex comprising:
(a) core layer of cerium oxide nanoparticles;
(b) an outer layer comprising a polymer represented by the following Formula 1: 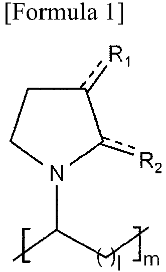 wherein R₁ and R₂ are each independently hydrogen or oxygen, - - - represents a single bond or a double bond, l is 1 or 2, and m is an integer of 100 to 1000.

2. The nanocomplex of claim 1, wherein the cerium oxide nanoparticles are selected from the group consisting of cerium (III) oxide (Ce₂O₃) nanoparticles, cerium (IV) oxide (CeO₂) nanoparticles, and a mixture thereof.

3. The nanocomplex of claim 1, wherein in Formula 1, R₁ is hydrogen, R₂ is oxygen, and 1 is 1.

4. The nanocomplex of claim 3, wherein the polymer represented by formula 1 and the cerium oxide nanoparticles have a content ratio of 7:1 to 11:1.

5. The nanocomplex of claim 1, further comprising a multifunctional ligand represented by the following Formula 2: 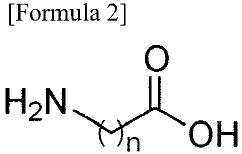 wherein n is an integer of 3 to 7.

6. The nanocomplex of claim 5, wherein in Formula 2, n is 5.

7. The nanocomplex of claim 1, wherein the nanocomplex has an average particle size of 5 nm to 80 nm.

8. The nanocomplex of claim 1, wherein the nanocomplex has a surface charge (Zeta potential) of -1.0 to 1.0 mV.

9. A composition for preventing or treating inflammatory or autoimmune diseases, comprising the nanocomplex of any one of claims 1 to 8 as an active ingredient.

10. The composition of claim 9, wherein the inflammatory or autoimmune diseases are one or more diseases selected from the group consisting of intracerebral hemorrhage (ICH), cerebral infarction, dermatitis, traumatic brain injury, inflammatory bowel disease (IBD), subarachnoid hemorrhage (SAH), cerebral edema, hypoxic ischemic encephalopathy, stroke, traumatic spinal cord injury, gunshot injury, acute respiratory distress syndrome, cytokine storm syndrome, sepsis, systemic inflammatory response syndrome, peritonitis, multiple sclerosis, rheumatoid arthritis, reactive arthritis, type 1 diabetes, systemic lupus erythematosus, idiopathic pulmonary fibrosis, polymyositis, dermatomyositis, localized scleroderma, systemic scleroderma, Sjogren's syndrome, Raynaud's phenomenon, Bechet's disease, Kawasaki's disease, primary biliary sclerosis, primary sclerosing cholangitis, psoriasis, myasthenia gravis, autoimmune vasculitis, primary angiitis of the central nervous system, and inflammatory liver disease.

11. The composition of claim 10, wherein the inflammatory or autoimmune disease is intracerebral hemorrhage (ICH).

12. The composition of claim 10, wherein the inflammatory or autoimmune disease is cerebral infarction.

13. The composition of claim 10, wherein the inflammatory or autoimmune disease is dermatitis.

14. The composition of claim 10, wherein the inflammatory or autoimmune disease is traumatic brain injury.

15. The composition of claim 10, wherein the inflammatory or autoimmune disease is inflammatory bowel disease (IBD).

16. The composition of claim 10, wherein the inflammatory or autoimmune disease is subarachnoid hemorrhage (SAH).

17. The composition of claim 10, wherein the inflammatory or autoimmune disease is sepsis.

18. The composition of claim 10, wherein the inflammatory or autoimmune disease is inflammatory liver disease.

19. A method for preparing a cerium oxide nanocomplex, comprising:
(a) preparing a mixed solution by adding a cerium precursor, a polymer represented by the following Formula 1, and a crosslinking compound represented by the following Formula 2 to a C₁-C₃ alcoholic solvent; 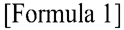 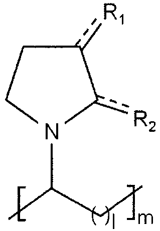
wherein R₁ and R₂ are each independently hydrogen or oxygen, - - - represents a single bond or a double bond, l is 1 or 2, and m is an integer of 100 to 1000; 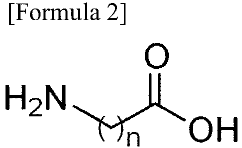
wherein n is an integer of 3 to 7;
(b) sequentially heating and cooling the mixed solution to obtain cerium oxide nanoparticles; and
(c) washing the cerium oxide nanoparticles with an inorganic salt solution.

20. The method of claim 19, wherein the C₁-C₃ alcohol solvent is ethanol.

21. The method of claim 19, wherein the cerium precursor is one or more precursors selected from the group consisting of cerium (III) acetate hydrate, cerium (III) acetylacetonate hydrate, cerium (III) carbonate hydrate, cerium (III) fluoride, cerium (III) chloride, cerium (III) chloride heptahydrate, cerium (III) bromide, cerium (III) iodide, cerium (III) nitrate hexahydrate, cerium (III) oxalate hydrate, cerium (III) sulfate, and cerium (III) sulfate hydrate.

22. The method of claim 19, wherein the heating is performed at 60 to 80°C.

23. The method of claim 19, wherein the cooling is performed by lowering the temperature to 35 to 50°C.

24. The method of claim 19, wherein the inorganic salt is selected from the group consisting of NaCl, CaCl₂, KCl, MgCl₂ and combinations thereof.

25. The method of claim 19, wherein the inorganic salt solution is NaCl solution.

26. The method of claim 25, wherein the inorganic salt solution has a concentration of 0.15 to 0.35 M.
